(19) Europäisches Patentamt
European Patent Office
Office européen des brevets

(11) **EP 3 177 647 B1**

(12) **EUROPEAN PATENT SPECIFICATION**

(45) Date of publication and mention of the grant of the patent:
**10.10.2018 Bulletin 2018/41**

(21) Application number: **15757443.5**

(22) Date of filing: **06.08.2015**

(51) Int Cl.:
**C07K 16/28** [(2006.01)]    **A61P 35/00** [(2006.01)]

(86) International application number:
**PCT/EP2015/068178**

(87) International publication number:
**WO 2016/020483 (11.02.2016 Gazette 2016/06)**

(54) **ANTI-HERG1 ANTIBODIES**

ANTI-HERG1-ANTIKÖRPER

ANTICORPS ANTI-HERG1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.08.2014  IT FI20140189**

(43) Date of publication of application:
**14.06.2017  Bulletin 2017/24**

(73) Proprietors:
- **Universita' Degli Studi di Firenze**
  **50121 Firenze (IT)**
- **Azienda Ospedaliero-Universitaria Careggi**
  **50134 Firenze (IT)**

(72) Inventors:
- **ARCANGELI, Annarosa**
  **I-50127 Firenze (IT)**
- **CROCIANI, Olivia**
  **I-50126 Firenze (IT)**
- **CRESCIOLI, Silvia**
  **I-50141 Firenze (IT)**
- **SETTE, Angelica**
  **I-71016 San Severo (IT)**

(74) Representative: **Valenza, Silvia et al**
**Notarbartolo & Gervasi S.p.A.**
**Corso di Porta Vittoria 9**
**20122 Milano (IT)**

(56) References cited:
**WO-A1-2011/058509    IT-A1- FI20 060 008**

- **Epigentek: "HERG1 monoclonal antibody a12", , 1 January 2015 (2015-01-01), XP002739553, Retrieved from the Internet: URL:http://www.epigentek.com/catalog/herg1-monoclonal-antibody-a12-p-3957.html?currency=gb&height=190&width=500&border=1&modal =true&random=1431503689546 [retrieved on 2015-04-15]**
- **LASTRAIOLI E ET AL: "herg1 gene and HERG1 protein are overexpressed in colorectal cancers and regulate cell invasion of tumor cells", CANCER RESEARCH, AMERICAN ASSOCIATION FOR CANCER RESEARCH, US, vol. 64, no. 2, 15 January 2004 (2004-01-15), pages 606-611, XP002383047, ISSN: 0008-5472, DOI: 10.1158/0008-5472.CAN-03-2360**
- **OLIVIA CROCIANI ET AL: "hERG1 channels modulate integrin signaling to trigger angiogenesis and tumor progression in colorectal cancer", SCIENTIFIC REPORTS, vol. 3, 25 November 2013 (2013-11-25), XP055183172, DOI: 10.1038/srep03308**
- **ARCANGELI A ET AL: "New trends in cancer therapy: Targeting ion channels and transporters", PHARMACEUTICALS 2010 MOLECULAR DIVERSITY PRESERVATION INTERNATIONAL CHE, vol. 3, no. 4, 2010, pages 1202-1224, XP002739554, ISSN: 1424-8247**
- **GEORG J. HAUSAMMANN ET AL: "Generation of an antibody toolbox to characterize hERG", BIOCHEMICAL AND BIOPHYSICAL RESEARCH COMMUNICATIONS, vol. 431, no. 1, 1 February 2013 (2013-02-01), pages 70-75, XP055183645, ISSN: 0006-291X, DOI: 10.1016/j.bbrc.2012.12.089**

- JAKUB SROUBEK ET AL: "The use of Bcl-2 over-expression to stabilize hybridomas specific to the HERG potassium channel", JOURNAL OF IMMUNOLOGICAL METHODS, ELSEVIER SCIENCE PUBLISHERS B.V.,AMSTERDAM, NL, vol. 375, no. 1, 26 October 2011 (2011-10-26), pages 215-222, XP028434312, ISSN: 0022-1759, DOI: 10.1016/J.JIM.2011.10.014 [retrieved on 2011-11-15]

- ELENA LASTRAIOLI ET AL: "hERG1 behaves as biomarker of progression to adenocarcinoma in Barrett's esophagus and can be exploited for a novel endoscopic surveillance", ONCOTARGET, vol. 7, no. 37, 13 September 2016 (2016-09-13), XP055458511, United States ISSN: 1949-2553, DOI: 10.18632/oncotarget.11149

SEQUENCE LISTING

<110> AZIENDA OSPEDALIERO-UNIVERSITARIA CAREGGI UNIVERSITA' DEGLI STUDI DI FIRENZE

<120> ANTI-hERG1 MOLECULES

<130> 12696PTWO

<150> ITFI2014A000189
<151> 2014-08-08

<160> 17

<170> BiSSAP 1.3

<210> 1
<211> 381
<212> DNA
<213> Mus musculus

<220>
<223> /note="anti-hERG1 VH" /organelle="mitochondrion"

<220>
<221> CDS
<222> 1..381
<223> /transl_table=2

<400> 1

```
gag gtc caa ctg caa cag tct gga cct gaa ctg gtg aag cct ggg gct        48
Glu Val Gln Leu Gln Gln Ser Gly Pro Glu Leu Val Lys Pro Gly Ala
1               5                   10                  15

tct gtg aag ata tcc tgc aag act tca gga tac aca ttc act gaa tac        96
Ser Val Lys Met Ser Cys Lys Thr Ser Gly Tyr Thr Phe Thr Glu Tyr
                20                  25                  30

acc gtt cac tgg gtg aaa cag agc cat gga aag agc ctt gaa tgg att       144
Thr Val His Trp Val Lys Gln Ser His Gly Lys Ser Leu Glu Trp Ile
            35                  40                  45

gga ggc att aat cct aat ggt ggt act acc tat aat cag aag ttc aag       192
Gly Gly Ile Asn Pro Asn Gly Gly Thr Thr Tyr Asn Gln Lys Phe Lys
        50                  55                  60

ggc aag gcc aca ttg act att gac aag tcc tcc agc tca gcc ttc atg       240
Gly Lys Ala Thr Leu Thr Ile Asp Lys Ser Ser Ser Ser Ala Phe Met
65                  70                  75                  80

gag ctc cgc agc ctg aca tct gag gat tct gca gtc tat tac ttt gca       288
Glu Leu Arg Ser Leu Thr Ser Glu Asp Ser Ala Val Tyr Tyr Phe Ala
                85                  90                  95

aca ggt tgg gga cct gac tac tgg ggc caa ggc acc act ctc aca gtc       336
Thr Gly Trp Gly Pro Asp Tyr Trp Gly Gln Gly Thr Thr Leu Thr Val
                100                 105                 110

tcc tca gcc aaa aca aca ccc cca tca gtc tat cca ctg gcc cct          381
Ser Ser Ala Lys Thr Thr Pro Pro Ser Val Tyr Pro Leu Ala Pro
            115                 120                 125
```

<210> 2
<211> 363
<212> DNA
<213> Mus musculus

<220>
<223> VL chain

<220>
<221> CDS
<222> 1..363
<223> /transl_table=1

<400> 2

```
gat att gtg ctg aca caa tct cca ctc act ttg tcg gtt aac att ggt      48
Asp Ile Val Leu Thr Gln Ser Pro Leu Thr Leu Ser Val Asn Ile Gly
1                   5                  10                  15

caa cca gcc tct atc tct tgc aag tca agt cag agc ctc tta tat act      96
Gln Pro Ala Ser Ile Ser Cys Lys Ser Ser Gln Ser Leu Leu Tyr Thr
            20                  25                  30

aat gga aaa acc tat ttt aat tgg tta tta cag agg cca ggc cag tct     144
Asn Gly Lys Thr Tyr Phe Asn Trp Leu Leu Gln Arg Pro Gly Gln Ser
        35                  40                  45

cca aag cgc cta atc tat ctg gtg tct aaa ctg gac tct gga gtc cct     192
Pro Lys Arg Leu Ile Tyr Leu Val Ser Lys Leu Asp Ser Gly Val Pro
    50                  55                  60

gac agg ttc act ggc agt gga tca gga aca gat ttt aca ctg aaa atc     240
Asp Arg Phe Thr Gly Ser Gly Ser Gly Thr Asp Phe Thr Leu Lys Ile
65                  70                  75                  80

agc aga gtg gag gct gaa gat ttg gga gtt tat tac tgc gcg caa ggt     288
Ser Arg Val Glu Ala Glu Asp Leu Gly Val Tyr Tyr Cys Ala Gln Gly
                85                  90                  95

aca cat ttt ccg tgg acg ttc ggt gga ggg acc aag ctg gaa atc aaa     336
Thr His Phe Pro Trp Thr Phe Gly Gly Gly Thr Lys Leu Glu Ile Lys
            100                 105                 110

cgg gct gat gct gca cca act gta tcc                                 363
Arg Ala Asp Ala Ala Pro Thr Val Ser
            115                 120
```

<210> 3
<211> 127
<212> PRT
<213> Mus musculus

<220>
<223> [CDS]:1..381 from SEQ ID NO 1

<400> 3

```
Glu Val Gln Leu Gln Gln Ser Gly Pro Glu Leu Val Lys Pro Gly Ala
1                   5                  10                  15
Ser Val Lys Met Ser Cys Lys Thr Ser Gly Tyr Thr Phe Thr Glu Tyr
            20                  25                  30
Thr Val His Trp Val Lys Gln Ser His Gly Lys Ser Leu Glu Trp Ile
            35                  40                  45
Gly Gly Ile Asn Pro Asn Gly Gly Thr Thr Tyr Asn Gln Lys Phe Lys
        50                  55                  60

Gly Lys Ala Thr Leu Thr Ile Asp Lys Ser Ser Ser Ser Ala Phe Met
65                  70                  75                  80
Glu Leu Arg Ser Leu Thr Ser Glu Asp Ser Ala Val Tyr Tyr Phe Ala
                85                  90                  95
Thr Gly Trp Gly Pro Asp Tyr Trp Gly Gln Gly Thr Thr Leu Thr Val
            100                 105                 110
Ser Ser Ala Lys Thr Thr Pro Pro Ser Val Tyr Pro Leu Ala Pro
            115                 120                 125
```

<210> 4
<211> 121
<212> PRT
<213> Mus musculus

<220>
<223> [CDS]:1..363 from SEQ ID NO 2

<400> 4

```
Asp Ile Val Leu Thr Gln Ser Pro Leu Thr Leu Ser Val Asn Ile Gly
1               5                   10                  15
Gln Pro Ala Ser Ile Ser Cys Lys Ser Ser Gln Ser Leu Leu Tyr Thr
            20                  25                  30
Asn Gly Lys Thr Tyr Phe Asn Trp Leu Leu Gln Arg Pro Gly Gln Ser
            35                  40                  45
Pro Lys Arg Leu Ile Tyr Leu Val Ser Lys Leu Asp Ser Gly Val Pro
        50                  55                  60
Asp Arg Phe Thr Gly Ser Gly Ser Gly Thr Asp Phe Thr Leu Lys Ile
65                  70                  75                  80
Ser Arg Val Glu Ala Glu Asp Leu Gly Val Tyr Tyr Cys Ala Gln Gly
                85                  90                  95
Thr His Phe Pro Trp Thr Phe Gly Gly Gly Thr Lys Leu Glu Ile Lys
            100                 105                 110
Arg Ala Asp Ala Ala Pro Thr Val Ser
            115                 120
```

<210> 5
<211> 798
<212> DNA
<213> Artificial Sequence

<220>
<223> scFv-hERG1

<220>
<221> CDS
<222> 1..798
<223> /note="scFV-hERG1" /transl_table=1

<400> 5

```
gag gtc caa ctg caa cag tct gga cct gaa ctg gtg aag cct ggg gct    48
Glu Val Gln Leu Gln Gln Ser Gly Pro Glu Leu Val Lys Pro Gly Ala
1               5                   10                  15

tct gtg aag ata tcc tgc aag act tca gga tac aca ttc act gaa tac    96
Ser Val Lys Ile Ser Cys Lys Thr Ser Gly Tyr Thr Phe Thr Glu Tyr
                20                  25                  30

acc gtt cac tgg gtg aaa cag agc cat gga aag agc ctt gaa tgg att   144
Thr Val His Trp Val Lys Gln Ser His Gly Lys Ser Leu Glu Trp Ile
            35                  40                  45

gga ggc att aat cct aat ggt ggt act acc tat aat cag aag ttc aag   192
```

```
Gly Gly Ile Asn Pro Asn Gly Gly Thr Thr Tyr Asn Gln Lys Phe Lys
    50                  55                  60

ggc aag gcc aca ttg act att gac aag tcc tcc agc tca gcc ttc atg    240
Gly Lys Ala Thr Leu Thr Ile Asp Lys Ser Ser Ser Ser Ala Phe Met
65                  70                  75                  80

gag ctc cgc agc ctg aca tct gag gat tct gca gtc tat tac ttt gca    288
Glu Leu Arg Ser Leu Thr Ser Glu Asp Ser Ala Val Tyr Tyr Phe Ala
                85                  90                  95

aca ggt tgg gga cct gac tac tgg ggc caa ggc acc act ctc aca gtc    336
Thr Gly Trp Gly Pro Asp Tyr Trp Gly Gln Gly Thr Thr Leu Thr Val
            100                 105                 110

tcc tca gcc aaa aca aca ccc cca tca gtc tat cca ctg gcc cct ggc    384
Ser Ser Ala Lys Thr Thr Pro Pro Ser Val Tyr Pro Leu Ala Pro Gly
            115                 120                 125

tcg agt ggt gga ggc ggt tca ggc gga ggt ggc tct ggc ggt agt gca    432
Ser Ser Gly Gly Gly Gly Ser Gly Gly Gly Gly Ser Gly Gly Ser Ala
        130                 135                 140

ctg gat att gtg ctg aca caa tct cca ctc act ttg tcg gtt aac att    480
Leu Asp Ile Val Leu Thr Gln Ser Pro Leu Thr Leu Ser Val Asn Ile
145                 150                 155                 160

ggt caa cca gcc tct atc tct tgc aag tca agt cag agc ctc tta tat    528
Gly Gln Pro Ala Ser Ile Ser Cys Lys Ser Ser Gln Ser Leu Leu Tyr
                165                 170                 175

act aat gga aaa acc tat ttt aat tgg tta tta cag agg cca ggc cag    576
Thr Asn Gly Lys Thr Tyr Phe Asn Trp Leu Leu Gln Arg Pro Gly Gln
            180                 185                 190

tct cca aag cgc cta atc tat ctg gtg tct aaa ctg gac tct gga gtc    624
Ser Pro Lys Arg Leu Ile Tyr Leu Val Ser Lys Leu Asp Ser Gly Val
            195                 200                 205

cct gac agg ttc act ggc agt gga tca gga aca gat ttt aca ctg aaa    672
Pro Asp Arg Phe Thr Gly Ser Gly Ser Gly Thr Asp Phe Thr Leu Lys
            210                 215                 220

atc agc aga gtg gag gct gaa gat ttg gga gtt tat tac tgc gcg caa    720
Ile Ser Arg Val Glu Ala Glu Asp Leu Gly Val Tyr Tyr Cys Ala Gln
225                 230                 235                 240

ggt aca cat ttt ccg tgg acg ttc ggt gga ggg acc aag ctg gaa atc    768
Gly Thr His Phe Pro Trp Thr Phe Gly Gly Gly Thr Lys Leu Glu Ile
                245                 250                 255

aaa cgg gct gat gct gca cca act gta tcc                            798
Lys Arg Ala Asp Ala Ala Pro Thr Val Ser
            260                 265
```

<210> 6
<211> 266
<212> PRT
<213> Artificial Sequence

<220>

<223> [CDS]:1..798 from SEQ ID NO 5

<400> 6

```
Glu Val Gln Leu Gln Gln Ser Gly Pro Glu Leu Val Lys Pro Gly Ala
1               5                   10                  15
Ser Val Lys Ile Ser Cys Lys Thr Ser Gly Tyr Thr Phe Thr Glu Tyr
            20                  25                  30
Thr Val His Trp Val Lys Gln Ser His Gly Lys Ser Leu Glu Trp Ile
            35                  40                  45
Gly Gly Ile Asn Pro Asn Gly Gly Thr Thr Tyr Asn Gln Lys Phe Lys
        50                  55                  60
Gly Lys Ala Thr Leu Thr Ile Asp Lys Ser Ser Ser Ala Phe Met
65                  70                  75                  80
Glu Leu Arg Ser Leu Thr Ser Glu Asp Ser Ala Val Tyr Tyr Phe Ala
                85                  90                  95
Thr Gly Trp Gly Pro Asp Tyr Trp Gly Gln Gly Thr Thr Leu Thr Val
            100                 105                 110
Ser Ser Ala Lys Thr Thr Pro Pro Ser Val Tyr Pro Leu Ala Pro Gly
            115                 120                 125
Ser Ser Gly Gly Gly Gly Ser Gly Gly Gly Gly Ser Gly Gly Ser Ala
        130                 135                 140
Leu Asp Ile Val Leu Thr Gln Ser Pro Leu Thr Leu Ser Val Asn Ile
145                 150                 155                 160
Gly Gln Pro Ala Ser Ile Ser Cys Lys Ser Ser Gln Ser Leu Leu Tyr
                165                 170                 175
Thr Asn Gly Lys Thr Tyr Phe Asn Trp Leu Leu Gln Arg Pro Gly Gln
            180                 185                 190
Ser Pro Lys Arg Leu Ile Tyr Leu Val Ser Lys Leu Asp Ser Gly Val
            195                 200                 205
Pro Asp Arg Phe Thr Gly Ser Gly Ser Gly Thr Asp Phe Thr Leu Lys
        210                 215                 220
Ile Ser Arg Val Glu Ala Glu Asp Leu Gly Val Tyr Tyr Cys Ala Gln
225                 230                 235                 240
Gly Thr His Phe Pro Trp Thr Phe Gly Gly Gly Thr Lys Leu Glu Ile
                245                 250                 255
Lys Arg Ala Asp Ala Ala Pro Thr Val Ser
                260                 265
```

<210> 7
<211> 24
<212> DNA
<213> Artificial Sequence

<220>
<223> degKappadir

<400> 7
gayattgtgm tsacmcarwc tmca          24

<210> 8
<211> 21
<212> DNA
<213> Artificial Sequence

<220>
<223> Kapparev

<400> 8
ggatacagtt ggtgcagcat c          21

```
<210> 9
<211> 20
<212> DNA
<213> Artificial Sequence

<220>
<223> degH2dir

<400> 9
gaggtccarc tgcaacartc          20

<210> 10
<211> 24
<212> DNA
<213> Artificial Sequence

<220>
<223> IgG2brev

<400> 10
aggggccagt ggatagactg atgg          24

<210> 11
<211> 30
<212> DNA
<213> Artificial Sequence

<220>
<223> VH for Sal I

<400> 11
acgcgtcgac gaggtccaac tgcaacagtc          30

<210> 12
<211> 35
<212> DNA
<213> Artificial Sequence

<220>
<223> VH rev Xho I

<400> 12
ccgctcgagc caggggccag tggatagact gatgg          35

<210> 13
<211> 36
<212> DNA
<213> Artificial Sequence

<220>
<223> VL for ApaL I

<400> 13
acgcgtgcac tggatattgt gctgacacaa tctcca          36

<210> 14
<211> 37
<212> DNA
```

<213> Artificial Sequence

<220>
<223> VL rev Not I

<400> 14
ataagaatgc ggccgcggat acagttggtg cagcatc         37

<210> 15
<211> 1101
<212> DNA
<213> Artificial Sequence

<220>
<223> scFv-hERG1 expression cassette

<220>
<221> CDS
<222> 1..1095
<223> /product="expressed protein" /transl_table=1

<400> 15

```
atg aga ttt cct tca att ttt act gca gtt tta ttc gca gca tcc tcc        48
Met Arg Phe Pro Ser Ile Phe Thr Ala Val Leu Phe Ala Ala Ser Ser
1               5                   10                  15

gca tta gct gct cca gtc aac act aca aca gaa gat gaa acg gca caa        96
Ala Leu Ala Ala Pro Val Asn Thr Thr Thr Glu Asp Glu Thr Ala Gln
                20                  25                  30

att ccg gct gaa gct gtc atc ggt tac tca gat tta gaa ggg gat ttc       144
Ile Pro Ala Glu Ala Val Ile Gly Tyr Ser Asp Leu Glu Gly Asp Phe
            35                  40                  45

gat gtt gct gtt ttg cca ttt tcc aac agc aca aat aac ggg tta ttg       192
Asp Val Ala Val Leu Pro Phe Ser Asn Ser Thr Asn Asn Gly Leu Leu
        50                  55                  60

ttt ata aat act act att gcc agc att gct gct aaa gaa gaa ggg gta       240
Phe Ile Asn Thr Thr Ile Ala Ser Ile Ala Ala Lys Glu Glu Gly Val
65                  70                  75                  80

tct ctc gag aaa aga gag gct gag gca gag gtc caa ctg caa cag tct       288
Ser Leu Glu Lys Arg Glu Ala Glu Ala Glu Val Gln Leu Gln Gln Ser
                85                  90                  95

gga cct gaa ctg gtg aag cct ggg gct tct gtg aag ata tcc tgc aag       336
Gly Pro Glu Leu Val Lys Pro Gly Ala Ser Val Lys Ile Ser Cys Lys
                100                 105                 110

act tca gga tac aca ttc act gaa tac acc gtt cac tgg gtg aaa cag       384
Thr Ser Gly Tyr Thr Phe Thr Glu Tyr Thr Val His Trp Val Lys Gln
            115                 120                 125

agc cat gga aag agc ctt gaa tgg att gga ggc att aat cct aat ggt       432
Ser His Gly Lys Ser Leu Glu Trp Ile Gly Gly Ile Asn Pro Asn Gly
        130                 135                 140

ggt act acc tat aat cag aag ttc aag ggc aag gcc aca ttg act att       480
Gly Thr Thr Tyr Asn Gln Lys Phe Lys Gly Lys Ala Thr Leu Thr Ile
145                 150                 155                 160

gac aag tcc tcc agc tca gcc ttc atg gag ctc cgc agc ctg aca tct       528
Asp Lys Ser Ser Ser Ser Ala Phe Met Glu Leu Arg Ser Leu Thr Ser
```

```
                 165                     170                     175

     gag gat tct gca gtc tat tac ttt gca aca ggt tgg gga cct gac tac      576
     Glu Asp Ser Ala Val Tyr Tyr Phe Ala Thr Gly Trp Gly Pro Asp Tyr
                 180                 185                 190

     tgg ggc caa ggc acc act ctc aca gtc tcc tca gcc aaa aca aca ccc      624
     Trp Gly Gln Gly Thr Thr Leu Thr Val Ser Ser Ala Lys Thr Thr Pro
                 195                 200                 205

     cca tca gtc tat cca ctg gcc cct ggc tcg agt ggt gga ggc ggt tca      672
     Pro Ser Val Tyr Pro Leu Ala Pro Gly Ser Ser Gly Gly Gly Gly Ser
                 210                 215                 220

     ggc gga ggt ggc tct ggc ggt agt gca ctg gat att gtg ctg aca caa      720
     Gly Gly Gly Gly Ser Gly Gly Ser Ala Leu Asp Ile Val Leu Thr Gln
     225                 230                 235                 240

     tct cca ctc act ttg tcg gtt aac att ggt caa cca gcc tct atc tct      768
     Ser Pro Leu Thr Leu Ser Val Asn Ile Gly Gln Pro Ala Ser Ile Ser
                     245                 250                 255

     tgc aag tca agt cag agc ctc tta tat act aat gga aaa acc tat ttt      816
     Cys Lys Ser Ser Gln Ser Leu Leu Tyr Thr Asn Gly Lys Thr Tyr Phe
                 260                 265                 270

     aat tgg tta tta cag agg cca ggc cag tct cca aag cgc cta atc tat      864
     Asn Trp Leu Leu Gln Arg Pro Gly Gln Ser Pro Lys Arg Leu Ile Tyr
                 275                 280                 285

     ctg gtg tct aaa ctg gac tct gga gtc cct gac agg ttc act ggc agt      912
     Leu Val Ser Lys Leu Asp Ser Gly Val Pro Asp Arg Phe Thr Gly Ser
                 290                 295                 300

     gga tca gga aca gat ttt aca ctg aaa atc agc aga gtg gag gct gaa      960
     Gly Ser Gly Thr Asp Phe Thr Leu Lys Ile Ser Arg Val Glu Ala Glu
     305                 310                 315                 320

     gat ttg gga gtt tat tac tgc gcg caa ggt aca cat ttt ccg tgg acg     1008
     Asp Leu Gly Val Tyr Tyr Cys Ala Gln Gly Thr His Phe Pro Trp Thr
                     325                 330                 335

     ttc ggt gga ggg acc aag ctg gaa atc aaa cgg gct gat gct gca cca     1056
     Phe Gly Gly Gly Thr Lys Leu Glu Ile Lys Arg Ala Asp Ala Ala Pro
                 340                 345                 350

     act gta tcc cct agg cat cat cac cat cac cat cat cac taatag         1101
     Thr Val Ser Pro Arg His His His His His His His His
                 355                 360                 365
```

<210> 16
<211> 365
<212> PRT
<213> Artificial Sequence

<220>
<223> [CDS]:1..1095 from SEQ ID NO 15

<400> 16

```
Met Arg Phe Pro Ser Ile Phe Thr Ala Val Leu Phe Ala Ala Ser Ser
1               5                   10                  15
Ala Leu Ala Ala Pro Val Asn Thr Thr Thr Glu Asp Glu Thr Ala Gln
            20                  25                  30

Ile Pro Ala Glu Ala Val Ile Gly Tyr Ser Asp Leu Glu Gly Asp Phe
        35                  40                  45
Asp Val Ala Val Leu Pro Phe Ser Asn Ser Thr Asn Asn Gly Leu Leu
    50                  55                  60
Phe Ile Asn Thr Thr Ile Ala Ser Ile Ala Ala Lys Glu Glu Gly Val
65                  70                  75                  80
Ser Leu Glu Lys Arg Glu Ala Glu Ala Glu Val Gln Leu Gln Gln Ser
                85                  90                  95
Gly Pro Glu Leu Val Lys Pro Gly Ala Ser Val Lys Ile Ser Cys Lys
            100                 105                 110
Thr Ser Gly Tyr Thr Phe Thr Glu Tyr Thr Val His Trp Val Lys Gln
            115                 120                 125
Ser His Gly Lys Ser Leu Glu Trp Ile Gly Gly Ile Asn Pro Asn Gly
    130                 135                 140
Gly Thr Thr Tyr Asn Gln Lys Phe Lys Gly Lys Ala Thr Leu Thr Ile
145                 150                 155                 160
Asp Lys Ser Ser Ser Ser Ala Phe Met Glu Leu Arg Ser Leu Thr Ser
                165                 170                 175
Glu Asp Ser Ala Val Tyr Tyr Phe Ala Thr Gly Trp Gly Pro Asp Tyr
            180                 185                 190
Trp Gly Gln Gly Thr Thr Leu Thr Val Ser Ser Ala Lys Thr Thr Pro
            195                 200                 205
Pro Ser Val Tyr Pro Leu Ala Pro Gly Ser Ser Gly Gly Gly Gly Ser
    210                 215                 220
Gly Gly Gly Gly Ser Gly Gly Ser Ala Leu Asp Ile Val Leu Thr Gln
225                 230                 235                 240
Ser Pro Leu Thr Leu Ser Val Asn Ile Gly Gln Pro Ala Ser Ile Ser
            245                 250                 255
Cys Lys Ser Ser Gln Ser Leu Leu Tyr Thr Asn Gly Lys Thr Tyr Phe
            260                 265                 270
Asn Trp Leu Leu Gln Arg Pro Gly Gln Ser Pro Lys Arg Leu Ile Tyr
            275                 280                 285
Leu Val Ser Lys Leu Asp Ser Gly Val Pro Asp Arg Phe Thr Gly Ser
            290                 295                 300
Gly Ser Gly Thr Asp Phe Thr Leu Lys Ile Ser Arg Val Glu Ala Glu
305                 310                 315                 320
Asp Leu Gly Val Tyr Tyr Cys Ala Gln Gly Thr His Phe Pro Trp Thr
                325                 330                 335
Phe Gly Gly Gly Thr Lys Leu Glu Ile Lys Arg Ala Asp Ala Ala Pro
            340                 345                 350
Thr Val Ser Pro Arg His His His His His His His His
            355                 360                 365
```

<210> 17
<211> 280
<212> PRT
<213> Artificial Sequence

<220>
<223> secreted scFv-hERG1-6xHis

<400> 17

18

```
Glu Ala Glu Ala Glu Val Gln Leu Gln Gln Ser Gly Pro Glu Leu Val
1               5               10              15
Lys Pro Gly Ala Ser Val Lys Ile Ser Cys Lys Thr Ser Gly Tyr Thr
            20              25              30
Phe Thr Glu Tyr Thr Val His Trp Val Lys Gln Ser His Gly Lys Ser
        35              40              45
Leu Glu Trp Ile Gly Gly Ile Asn Pro Asn Gly Gly Thr Thr Tyr Asn
    50              55              60
Gln Lys Phe Lys Gly Lys Ala Thr Leu Thr Ile Asp Lys Ser Ser Ser
65              70              75              80
Ser Ala Phe Met Glu Leu Arg Ser Leu Thr Ser Glu Asp Ser Ala Val
                85              90              95
Tyr Tyr Phe Ala Thr Gly Trp Gly Pro Asp Tyr Trp Gly Gln Gly Thr
            100             105             110
Thr Leu Thr Val Ser Ser Ala Lys Thr Thr Pro Pro Ser Val Tyr Pro
        115             120             125
Leu Ala Pro Gly Ser Ser Gly Gly Gly Gly Ser Gly Gly Gly Gly Ser
    130             135             140
Gly Gly Ser Ala Leu Asp Ile Val Leu Thr Gln Ser Pro Leu Thr Leu
145             150             155             160
Ser Val Asn Ile Gly Gln Pro Ala Ser Ile Ser Cys Lys Ser Ser Gln
            165             170             175
Ser Leu Leu Tyr Thr Asn Gly Lys Thr Tyr Phe Asn Trp Leu Leu Gln
        180             185             190
Arg Pro Gly Gln Ser Pro Lys Arg Leu Ile Tyr Leu Val Ser Lys Leu
        195             200             205
Asp Ser Gly Val Pro Asp Arg Phe Thr Gly Ser Gly Ser Gly Thr Asp
        210             215             220
Phe Thr Leu Lys Ile Ser Arg Val Glu Ala Glu Asp Leu Gly Val Tyr
225             230             235             240
Tyr Cys Ala Gln Gly Thr His Phe Pro Trp Thr Phe Gly Gly Gly Thr
            245             250             255
Lys Leu Glu Ile Lys Arg Ala Asp Ala Ala Pro Thr Val Ser Pro Arg
        260             265             270
His His His His His His His His
        275             280
```

**Description**

FIELD OF THE INVENTION

The present invention relates to the field of antibodies, in particular it relates to anti-hERG1 molecules.

STATE OF THE ART

Over the past two decades, the antibodies' production technology has been significantly improved through antibody engineering; the advent of new technologies in the field of molecular engineering, led to the production of a wide variety of genetically engineered antibodies, such as fragments of type Fab, Fv form of simple chain of scFv, diabodies, triabodies, bispecifics, minibodies, phage antibodies (Holliger & Hudson, 2005). In fact there is a range of applications, in which the Fc-mediated effects are not required and even undesirable, because of their associated toxic effects and their capacity to evoke an immune response able to neutralise the antibody efficacy, when its Fc derived from a non human source.

Among the engineered antibody fragments, the Single Chain Variable Fragment (scFv) is the most popular and one of the smallest recombinant format with an antigen-binding activity function and with the property to be easily manageable for immunological application (Heng & Othman, 2006).

A scFv consists of variable regions of heavy (VH) and light (VL) chains, which are joined together by a flexible peptide linker, without compromising the fidelity of the VH-VL paring and antigen-binding sites. The choice of linker can affect the solubility, expression and correct folding of the scFv. Peptide linkers can vary from 10 to 25 amino acids in length and are typically, composed of hydrophilic amino acids such as glycine (G) and serine (S) (Shen et al., 2008). Hydrophilic sequences prevent intercalation of the peptide within or between the variable domains throughout the protein folding (Argos, 1990). The most common linker used is the (Gly4Ser)3 motif (Huston et al., 1988), due to its flexibility, neutral charge and solubility (Kortt et al., 2001). The use of scFv in diagnostics and therapy provides several advantages over whole antibodies, especially in solid tumours therapy; in fact the speed of penetration by a fragment versus an intact molecule is the most remarkable advantage. In 1988, it was established that an intact molecule of IgG took fifty-four hours to penetrate 1 mm into a solid tumour, while a Fab fragment managed the same distance in sixteen hours (Jain, 1987). Moreover, the scFv, as well as all the other antibody fragments format, can be forget into multivalent and multispecific reagents or easily linked to therapeutic tools as radionuclides, toxins or nanoparticles) and engineer to improve their diagnostic and therapeutic efficacy. hERG1 is a protein which is aberrantly expressed in tumours and other diseases (Arcangeli & Becchetti, 2010). Antibodies against hERG1 have a high potential application in diagnosis and therapy of a large variety of tumours and other diseases which are characterized by an over expression of hERG1.

In the Italian Patent IT1367861 is described a hybridoma cell line clone, named A7, able to secrete an anti-hERG1 monoclonal antibody (mAb) specific against the S5-pore extracellular portion of hERG1.

Aim of the present invention is to provide further antibodies against hERG1 in order to overcome some problems related to the *in vivo* use of murine complete antibodies (immunogenicity and big size of the molecule preventing an efficient permeability).

SUMMARY OF THE INVENTION

Herein is described the detailed structure of an intact murine monoclonal anti-hERG1 molecule and the corresponding anti-hERG1 scFv antibody production, obtained after the isolation of the mAb anti-hERG1 VH and VL. Such scFv has the same specificity of the correspondent whole antibody, and thus it is able to recognize the same hERG1 protein, aberrantly expressed in tumours and other diseases.

Subject-matter of the present invention is therefore a molecule comprising a Heavy chain Variable (VH) domain having SEQ ID NO:3 and a Light chain Variable domain having SEQ ID NO:4, said molecule having specificity against hERG1 S5-pore extracellular portion.

mAb-hERG1 was initially structurally well characterised and then engineered to produce a recombinant single chain variable fragment against hERG1 (scFv-hERG1). The main advantage of scFv over intact whole IgG was its small size; scFv dimensions allow it to penetrate more rapidly. In addition, the lack of constant regions decreased retention by Fc receptors found in most tissues and organs, which further reduced the side effects. These characteristics rendered scFv suitable for an in vivo administration and an ideal vector for delivery of agents such as radionuclide, enzyme, drugs or toxin. Moreover, it can be easily bound the different tools (es. nanoparticles, Kim et al., 2002) to produce bio-sensors or bio-system for diagnostic and therapeutic purposes. It is worth noting that the structurally characterization of the whole mAb-hERG1 open to the possibility to obtain any format of engineered antibody with a single or a double specificity.

DETAILED DESCRIPTION OF THE INVENTION

Molecule according to the invention can be a murine or fully humanized mAb. Molecule according to the invention can be a scFv or any other engineered antibody such as Fab, Fv form of simple chain of scFv, diabodies, triabodies, bispecifics, minibodies, phage antibodies.

A molecule according to the invention is useful as diagnostic or therapeutic tool.

Pathologies which can be diagnosed or treated using a molecule according to the invention are all those pathologies characterized by an over expression or mis-expression of hERG1 protein. Among said pathologies can be listed tumours, neurological diseases, endocrine diseases and neuro-endocrine diseases.

A molecule according to the invention, in particular the scFV, can also be used as a pharmaceutical delivery vector: so for example it can be covalently on not bonded to radionuclide, enzyme, drugs or toxin.

Further subject-matter of the present invention are therefore also pharmaceutical compositions comprising a molecule according to the invention.

A molecule according to the invention can be prepared by employing nucleotide sequences SEQ ID NO:1 and SEQ ID NO:2 encoding respectively VH (SEQ ID NO:3) and VL (SEQ ID NO:4).

Particularly preferred according to the invention is a method for preparing a scFv according to the invention, said method comprising the use of nucleotide sequence SEQ ID NO:5 encoding for a scFV having SEQ ID NO:6.

The method according to the invention implies recombinant techniques.

Therefore subject-matter of the present invention are also an expression vector or a plasmid comprising SEQ ID NO:1 and SEQ ID NO:2 as well as genetically modified microorganisms comprising an expression vector according to the invention.

The present invention could be better understood in light of the experimental section below.

BRIEF DESCRIPTION OF THE FIGURES

Fig.1. - Electrophoresis relative to PCR products of variable light (VL) and heavy (VH) chains.

Fig. 2. - VH(SEQ ID NO:1) (A) and VL(SEQ ID NO:2) (B) nucleotide sequences relative to the whole anti-hERG1 mAb. In the bottom panel the corresponding amino acid sequence (SEQ ID NO:3 and SEQ ID NO:4) with the CDR domains underlined is shown.

Fig. 3 - ScFv-hERG1 expression cassette sequence obtained by Automated DNA sequencing service (PRIMM). Underlined nucleotides represent the restriction sites used for the cloning of the CDS for scFv-hERG1 construct; in red bold the peptide linker sequence codifying a glycine-serine stretch (see below).

Fig. 4 - Amino acid sequence of scFv-hERG1. The highlighted sequences underlined show the scFv-hERG1 CDRs (heavy and light chain) of the recombinant antibody fragments. The framework regions are highlighted in grey and the glycine-serine linker region in bold-italics.

Fig. 5 - Histogram representing the signal obtained in ELISA against S5-Pore peptide testing the aliquot of Not Bound (NB), Wash (W) and Elution (E) obtained from Mut+ and MutS yeast supernatant purification. As expected, aliquot E is the most efficient one, having a higher concentration of scFv-hERG1.

Fig. 6 - Indirect immunofluorescence on hERG1 positive cell line PANC-1 using the scFv-hERG1 antibody. PANC-1 cells were grown on glass coverslips and fixed with 4% methanol-free formaldehyde (Thermo Scientific) in PBS. As we can see in

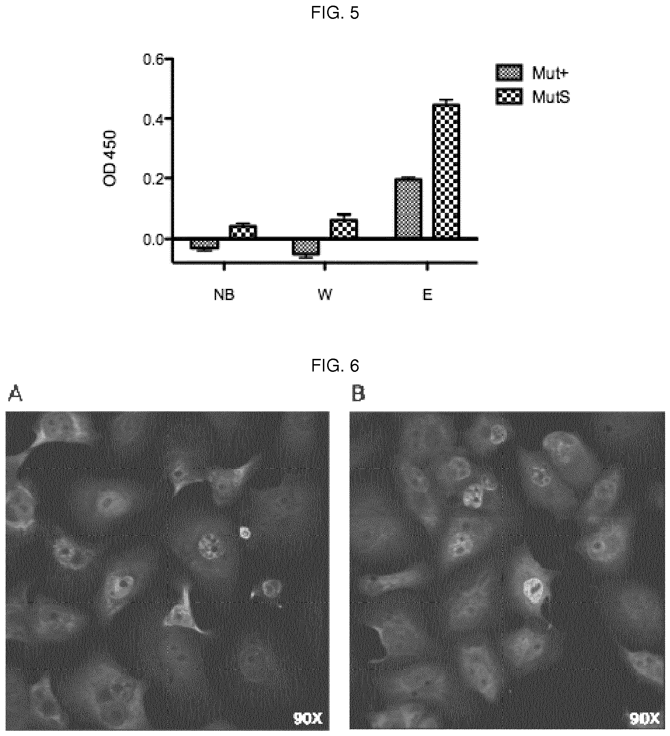

Figure 6, hERG1 scFv (B) has the same ability to bind hERG1 positive cells than the original mAb anti-hERG1 (A) from which the scFv was obtained.

EXPERIMENTAL SECTION

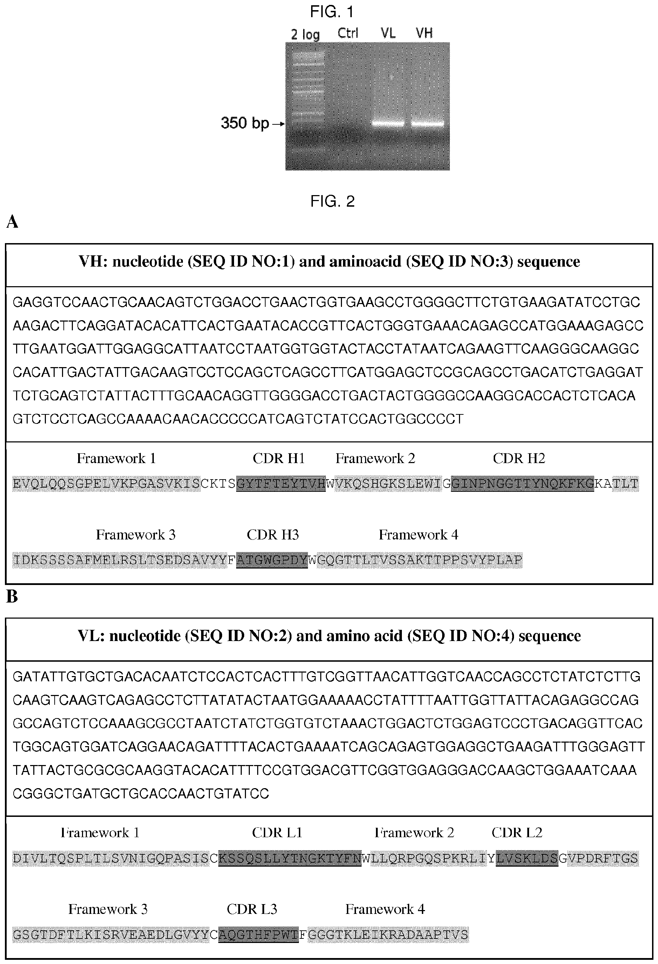

Initially it was determined the isotype of a mAb-hERG1 clone, named A21, able to secrete an anti-hERG1 with the same functional characteristics of the one described in IT1367861. The mAb-hERG1 resulted in a mouse IgG2b and this finding was the starting point to engineer such molecule to obtain different anti-hERG1 antibodies with different structures. To this purpose, we extracted and retro-transcribed the mRNA of the corresponding hybridoma, and were designed the primers SEQ ID NO:7-10 (listed in Materials and Methods paragraph 1.3 below) to obtain the VH and VL amplificates reported in Fig.1.

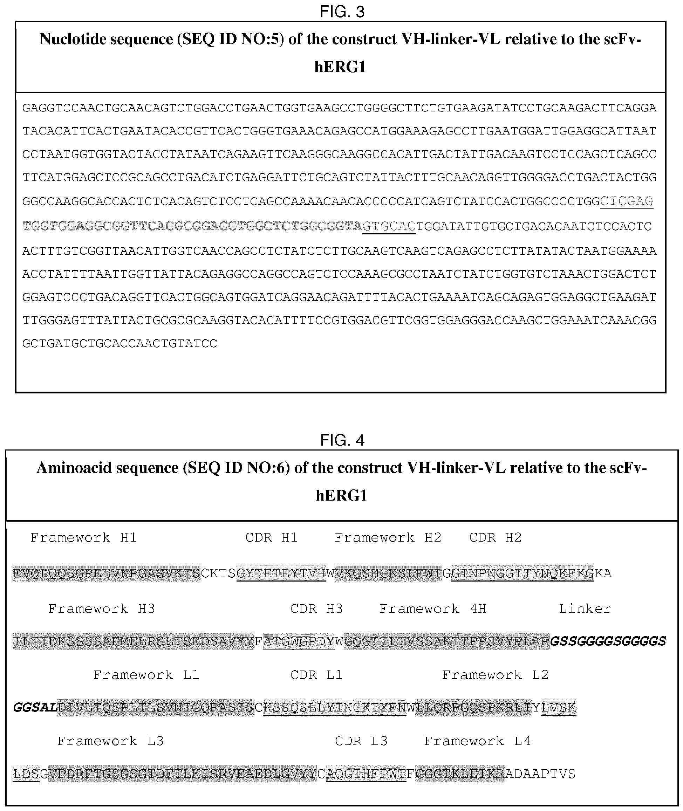

The purification of the bands and the relative ligation and transformation in DH5α bacteria strain, allowed to sequence the two fragments, which resulted from the analysis performed as described in Materials and Methods with the following nucleotide (SEQ ID NO: 1-2) and amino acid (SEQ ID NO:3-4) sequences (Fig.2). Then it followed the assembling VH and VL chains in the pHenIX vector. To this purpose, it was cloned VH between Sal I (SEQ ID NO:11) and Xho I (SEQ ID NO:12) and VL between ApaL I (SEQ ID NO:13) and Not I (SEQ ID NO:14). The sequence of the new construct obtained by this cloning is SEQ ID NO:5 reported in Fig. 3 Finally, once obtained the scFv-hERG1 construct (SEQ ID N. 15), the expression step was performed in Pichia Pastoris competent cells (as described in Materials and Methods paragraph 2.2 below). Both Mut+ and MutS strains were used and then compared for the scFv-hERG1 production.

Yeast supernatant, containing scFv-hERG1-6xHis (SEQ ID N: 17), was collected and concentrated with Amicon Ultra-15 10K (Millipore) and purified by chromatography using columns packed with Ni-NTA Agarose resin (Qiagen). Aliquots obtained from purification, marked as Not Bound (NB), Wash (W) and Elution (E), were analyzed through SDS-Page Comassie Staining and therefore by Western Blot, using an anti-6xHis antibody (GeneTex), to verify for the presence of the corresponding scFv-hERG1 (around 30 kDa). Once demonstrated an efficient production of the antibody, the following step was the evaluation of its ability to bind the antigen. To this purpose, it was performed an ELISA using hERG1 S5-Pore peptide coated plates (the same coating which was used for the whole mAb-hERG1 screening). The aliquots Not Bound (NB), Wash (W) and Elution (E), obtained from Mut+ and MutS yeast supernatant purification, were analysed.

## MATERIALS AND METHODS

### 1 Isolation of mAb anti-hERG1 (mAb-hERG1) heavy (VH) and light (VL) chain variable domains

#### 1.1 Determination of mAb-hERG1 isotype

To this purpose, the Mouse Monoclonal Antibody Isotyping Reagents (Sigma-Aldrich).

#### 1.2 mRNA extraction and retro-transcription

To obtain the mAb-hERG1 variable VH and VL chains able to recognise the hERG1 antigen expressed on the cellular membranes, we amplified the hybridoma cell line able to produce and secrete the mAb-hERG1 and total RNA was extracted using TRIzol® Reagent (Ambion), following the manifacturer's instructions. Then, messenger RNA (mRNA) was obtained using Poly(A)Purist® (Ambion).

mRNA was retro-transcribed into the corresponding cDNA encoding the VH and VL sequences using Random Primers (225 ng, final concentration/reaction) and SuperScript II Reverse Transcriptase (Invitrogen).

#### 1.3 PCR amplification

For the amplification of VH and VL regions, a 5' primer that anneals to the VH and VL framework 1 (FR1) (primer forward) and a primer that anneals to the constant region adjacent to VH and VL domains (primer reverse) were chosen. For VL, a degenerate primer able to recognise the kappa light chain was designed. For VH, a degenerate primer that anneals to the IgG2b heavy chain was designed. The primers used are described in Wang et al., 2000 and are reported below:

| NAME | SEQUENCE | SEQ ID NO: |
|------|----------|------------|
| degKappadir | GAYATTGTGMTSACMCARWCTMCA | 7 |
| Kapparev | GGATACAGTTGGTGCAGCATC | 8 |
| degH2dir | GAGGTCCARCTGCAACARTC | 9 |
| IgG2brev | AGGGGCCAGTGGATAGACTGATGG | 10 |

The amplification reaction of VH and VL was performed with the Phusion® High-Fidelity DNA Polymerase (Finnzymes Reagents), following the manifacturer's instructions, as described below.

| Steps Temperature | | Time |
|-------------------|------|------|
| 1 Initial denaturation minutes | 94°C | 2 |
| 2 Denaturation seconds | 94°C | 30 |
| 3 Annealing | 56°C (VH); 48°C (VL) | 1 minute |

(continued)

| Steps Temperature | | Time |
|---|---|---|
| 4 Extension | 72 °C | 1 minute |
| 5 Final extension | 72 °C | 10 minutes |

Steps 2,3 and 4 were repeated for 24 times.

### 1.4 *VH and VL cloning*

PCR products relative to VH and VL were run on 1% agarose in TAE buffer (Tris, acetic acid and EDTA), then excised from the gel with a scalpel and purified using QIAquick PCR Purification Kit (QIAGEN), according to the manufacturer's instruction. QIAquick Kits contain a silica membrane assembly for binding of DNA in high-salt buffer and elution with low-salt buffer or water. The purification procedure removes impurities from DNA samples.

After purification from agarose gel, PCR products were cloned into pCR™ -Blunt vector (where it is possible to insert blunt PCR products), using a 10:1 molar ratio of insert:vector, according to the following formula:

$$X \text{ ng insert} = (10)(Y \text{ bp PCR product)} (25 \text{ ng linearized pCR™ -Blunt})/(3500 \text{ bp pCR™ -Blunt})$$

where X ng is the amount of PCR product of Y base pairs to be ligated. The ligation reaction was incubated at 16°C for one hour.

DH5$\alpha$ cells were used for the transformation step and the selected colonies were checked for the presence of the insert in the right orientation.

### 1.5 *Sequencing and analysis of VH and VL*

DNA samples were sequenced by PRIMM s.r.l. and the relative products were analysed using ExPASy translation Tool software and basing on Kabat numbering scheme (www.bioinf.org.uk), in order to find the three scFv Complementary Determining Regions (CDR1, CDR2 and CDR3).

### 2 *Anti-hERG1 scFv development*

### 2.1 *scFv-hERG1 assembly*

To assembly VH and VL, in order to obtain the final scFv construct, phagemid vector pHenIX (Hoogenboom et al., 1991) and specific primers containing restriction enzyme recognition sites were used. Primer's sequences are shown below and restriction sites are reported as underlined. When needed, 2 additional bases (reported in blue) to preserve the sequence frame were included.

| NAME | SEQUENCE | SEQ ID NO: |
|---|---|---|
| VH for Sal I | 5' ACGCGTCGACGAGGTCCAACTGCAACAGTC | 11 |
| VH rev Xho I | 5' CCGCTCGAGCCAGGGGCCAGTGGATAGACTGATGG | 12 |
| VL for ApaL I | 5' ACGCGTGCACTGGATATTGTGCTGACACAATCTCCA | 13 |
| VL rev Not I | 5' ATAAGAATGCGGCCGCGGATACAGTTGGTGCAGCATC | 14 |

After PCR amplication and restriction enzyme cutting, sequences were cloned in the phagemid vector pHenIX using T4 DNA Ligase (New England Biolabs), following the manifacturer's instructions and basing on the formula reported in paragraph 1.4.

### 2.2 *scFv-hERG1 expression*

For the scFv-hERG1 expression, the yeast host Pichia Pastoris was used. To this purpose, we cloned the scFv-hERG1 construct having SEQ ID 15 into the commercial pPIC9K vector (kindly provided by Prof. E. Gherardi, University of Pavia), which has allowed us to express a 6xHis tagged protein in Pichia Pastoris. The expression cassette was amplified by PCR using primers containing the FspI and AvrII restriction sites at 3' and 5' ends, respectively. The expression cassette was then cutted with with FspI and AvrII and cloned into pPIC9K cutted with Eco53KI and AvrII restriction enzymes (NEB).

Pichia Pastoris competent cells was transformed by electroporation using 0.2 cm cuvette, 2000V, 400 ohm, 25 μF. Yeasts were then seeded on MD plates (Minimal Dextrose Medium: 1.34% YNB, 4x10$^{-5}$% biotin, 2% dextrose) + agar and incubated at 30 °C for 3 days. We decided to express scFv-hERG1 in both Mut+ or MutS strains according to Pichia Expression Kit (Invitrogen) protocol. A protein having SEQ ID N. 16 was expressed by the yeast. The protein having SEQ ID 16 was cut by the yeast at residue 85 before being secreted in the supernatant thus containing a scFv-hERG1-6xHis having SEQ ID N. 17 (corresponding to residues 86-365 of SEQ ID N. 16).

### 2.3 *ELISA assay*

In order to check for the scFv-hERG1 specificity, ELISA assay was performed on yeast supernatant purified aliquots obtained from Mut+ and MutS yeast following a standard protocol and using an anti-6xHis antibody (GeneTex) 1:500 in PBS + 3%BSA as primary antibody, followed by an anti-rabbit IgG-HRP conjugate antibody (Sigma) 1:500 in PBS + 3%BSA. As it can be seen in Figure 6, hERG1 scFv (B) has the same ability to bind hERG1 positive cells than the original mAb anti-hERG1 (A) from which the scFv was obtained.

### 2.4. Indirect immunofluorescence assay

To compare scFv-hERG1 and the full length antibody (hERG1 mAb) ability to bind hERG1 we performed and indirect immunofluorescence on hERG1 positive cell line PANC-1. PANC-1 cells were grown on glass coverslips and fixed with 4% methanol-free formaldehyde (Thermo Scientific) in PBS. Coverslips were incubated fifteen minutes in PBS 1% SDS at room temperature for antigen retrieval, treated fifteen minutes with 100 mM glycine at room temperature (to quench residual cross-linking activity of the formaldehyde) and permeabilised four minutes with PBS 0.01% Triton X-100. Unspecific binding sites were blocked thirty minutes at room temperature with PBS 10% FBS. To assay anti-hERG1 mAb binding, coverslips were incubated one hour at room temperature with anti-hERG1 mAb (1 μg/ml in PBS 10% FBS) followed by 45 minutes incubation at room temperature with Alexa-488 labelled antimouse antibody (Invitrogen) (1:500 in PBS 10% FBS). To assay scFv hERG1 binding, coverslips were incubated 2 hours at room temperature with scFv hERG1 (1:2 in PBS 10% FBS) . To reveal the scFv, coverslips were incubated 1 hour with anti-6xHis antibody (GeneTex) 1:500 in PBS 10% FBS, followed by 45 minutes incubation with Alexa-488 labelled anti-rabbit antibody (Invitrogen) (1:500 in PBS 10% FBS).

As we can see in Figure 6, hERG1 scFv (B) has the same ability to bind hERG1 positive cells than the full lenght antibody (A).

BIBLIOGRAFY

- Holliger P, Hudson PJ. 2005 Engineered antibody fragments and the rise of single domains. Nature Biotechnology 23, 1126 - 1136.
- Heng CK, Othman RY. 2006 Bioinformatics in molecular immunology laboratories demonstrated: Modeling an anti-CMV scFv antibody. Bioinformation 1: 118-120.
- Shen, Z, Yan H, Zhang Y, Mernaugh RL, Zeng X. 2008 Engineering peptide linkers for scFv immunosensors. Anal. Chem. 80: 1910-1917.
- Argos, P. (1990). An investigation of oligopeptides linking domains in protein tertiary structures and possible candidates for general gene fusion. J Mol Biol. 211: 943-958.
- Huston, J. S., D. Levinson, M. Mudgett-Hunter, M. S. Tai, J. Novotny, M. N. Margolies, R. J. Ridge, R. E. Bruccoleri, E. Haber, R. Crea and et al. 1988 Protein engineering of antibody binding sites: recovery of specific activity in an anti-digoxin single-chain Fv analogue produced in Escherichia coli. Proc. Natl. Acad. Sci. USA 85: 5879-5883.
- Kortt AA., Dolezal O, Power BE,Hudson PJ 2001 Dimeric and trimeric antibodies: high avidity scFvs for cancer targeting. Biomol Eng. 18: 95-108.
- Jain RK. 1987 Transport of molecules across tumor vasculature. Cancer Metastasis Rev. 6: 559-593.
- Arcangeli A, Becchetti A. 2010 New trends in cancer therapy: targeting ion channels and transporters. Pharmaceuticals, 3: 1202-1224.
- Wang, Z., Raifu M, Howard M, Smith L, Hansen D, Goldsby R, Ratner D 2000 Universal PCR amplification of mouse immunoglobulin gene variable regions: the design of degenerate primers and an assessment of the effect of DNA polymerase 3' to 5' exonuclease activity. J. Immunol. Methods 233: 167-177.

- Hoogenboom HR, Volckaert G, Raus JC 1991 Construction and expression of antibody-tumor necrosis factor fusion proteins. Mol. Immunol 28: 1027-1037.
- Kim BY, Rutka JT and Chan WC 2010 Nanomedicine. N. Engl. J. Med. 363: 2434-2443.
- Clark M. 2000 Antibody humanization: a case of the 'Emperor's new clothes'? Immunol Today. 21: 397-402.

**Claims**

1.  An anti-ERG1 molecule comprising a Heavy chain Variable (VH) domain having SEQ ID NO:3 and a Light chain Variable domain having SEQ ID NO:4, said molecule having specificity against hERG1 S5-pore extracellular portion, wherein said anti-ERG1 molecule is a Single Chain Variable Fragment (scFV).

2.  The molecule according to claim 1 which is an engineered antibody having one, two or more different antigen binding sites.

3.  The scFv according to claim 1 comprising SEQ ID NO:6.

4.  The molecule according to any one of claims 1-3 for use as a medicament.

5.  The molecule according to any one of claims 1-3 for use in the treatment and/or diagnosis of pathologies **characterised by** over expression of hERG1.

6.  The molecule for use according to claim 5 wherein the pathologies are selected in the group consisting of tumours, neurological diseases, endocrine diseases and neuro-endocrine diseases.

7.  A pharmaceutical composition comprising a molecule according to any one of claims 1-3.

8. A nucleotide sequence comprising SEQ ID NO:1 and SEQ ID NO:2 respectively encoding VH and VL according to claim 1.

9. Nucleotide sequence according to claim 8 and comprising SEQ ID NO:5.

10. An expression vector comprising the nucleotide sequences according to any one of claims 8-9.

11. A genetically-modified micro-organism comprising an expression vector according to claim 10.

12. A method for producing a molecule according to any one of claims 1-3 wherein nucleotide sequences according to any one of claims 8-9 are used.

13. A method for producing the scFv according to claim 3 wherein the nucleotide sequence SEQ ID NO:5, according to claim 9 is used.

**Patentansprüche**

1. Ein Anti-ERG1-Molekül, das eine variable Domäne einer schweren Kette (VH), die SEQ ID NO:3 aufweist, und eine variable Domäne einer leichten Kette, die SEQ ID NO:4 aufweist, umfasst, wobei das Molekül eine Spezifität gegenüber dem extrazellulären Teil von hERG1-S5-Pore aufweist, wobei das Anti-ERG1-Molekül ein variables Einzelkettenfragment (scFV) ist.

2. Das Molekül nach Anspruch 1, das ein gentechnisch hergestellter Antikörper mit einer, zwei oder mehr unterschiedlichen Antigenbindungsstelle(n) ist.

3. Das scFv nach Anspruch 1, das SEQ ID NO:6 umfasst.

4. Das Molekül nach einem der Ansprüche 1-3 zur Verwendung als Medikament.

5. Das Molekül nach einem der Ansprüche 1-3 zur Verwendung bei der Behandlung und/oder Diagnose von Pathologien, die durch eine Überexpression von hERG1 gekennzeichnet sind.

6. Das Molekül zur Verwendung nach Anspruch 5, wobei die Pathologien aus der Gruppe bestehend aus Tumoren, neurologischen Erkrankungen, endokrinen Erkrankungen und neuroendokrinen Erkrankungen ausgewählt sind.

7. Eine pharmazeutische Zusammensetzung, die ein Molekül nach einem der Ansprüche 1-3 umfasst.

8. Eine Nucleotidsequenz, umfassend SEQ ID NO:1 und SEQ ID NO:2, die jeweils für VH und VL nach Anspruch 1 kodiert.

9. Nucleotidsequenz nach Anspruch 8 und umfassend SEQ ID NO:5.

10. Ein Expressionsvektor, der die Nucleotidsequenzen nach einem der Ansprüche 8-9 umfasst.

11. Ein genetisch veränderter Mikroorganismus, der einen Expressionsvektor nach Anspruch 10 umfasst.

12. Ein Verfahren zur Herstellung eines Moleküls nach einem der Ansprüche 1-3, wobei Nucleotidsequenzen nach einem der Ansprüche 8-9 verwendet werden.

13. Ein Verfahren zur Herstellung des scFvs nach Anspruch 3, wobei die Nucleotidsequenz SEQ ID NO:5 nach Anspruch 9 verwendet wird.

**Revendications**

1. Une molécule anti-ERG1 comprenant un domaine variable à chaîne lourde (VH) ayant SEQ ID NO:3 et un domaine variable à chaîne légère ayant SEQ ID NO:4, ladite molécule ayant une spécificité contre la partie extracellulaire des pores S5 hERG1, dans laquelle ladite molécule anti-ERG1 est un fragment variable à chaîne unique (scFV).

2. La molécule selon la revendication 1, qui est un anticorps modifié ayant un, deux ou plusieurs sites de liaison d'antigène différents.

3. Le scFv selon la revendication 1 comprenant SEQ ID NO:6.

4. La molécule selon l'une quelconque des revendications 1 à 3 à utiliser comme médicament.

5. La molécule selon l'une quelconque des revendications 1 à 3, à utiliser dans le traitement et/ou le diagnostic de pathologies **caractérisées par** l'expression excessive de hERG1.

6. La molécule à utiliser selon la revendication 5, dans laquelle les pathologies sont sélectionnées dans le groupe comprenant des tumeurs, des maladies neurologiques, des maladies endocriniennes et des maladies neuroendocriniennes.

7. Une composition pharmaceutique comprenant une molécule selon l'une quelconque des revendications 1-3.

8. Une séquence nucléotidique comprenant SEQ ID NO:1 et SEQ ID NO:2 codant respectivement VH et VL selon la revendication 1.

9. Séquence nucléotidique selon la revendication 8 et comprenant SEQ ID NO:5.

10. Un vecteur d'expression comprenant les séquences nucléotidiques selon l'une quelconque des revendications 8-9.

11. Un micro-organisme génétiquement modifié comprenant un vecteur d'expression selon la revendication 10.

12. Un procédé de production d'une molécule selon l'une quelconque des revendications 1-3, dans lequel des séquences nucléotidiques selon l'une quelconque des revendications 8-9 sont utilisées.

13. Un procédé de production du scFv selon la revendication 3, dans lequel la séquence nucléotidique SEQ ID NO:5 selon la revendication 9 est utilisée.

FIG. 1

2 log   Ctrl   VL   VH

350 bp →

FIG. 2

**A**

| VH: nucleotide (SEQ ID NO:1) and aminoacid (SEQ ID NO:3) sequence |
|---|

GAGGTCCAACTGCAACAGTCTGGACCTGAACTGGTGAAGCCTGGGGGCTTCTGTGAAGATATCCTGC
AAGACTTCAGGATACACATTCACTGAATACACCGTTCACTGGGTGAAACAGAGCCATGGAAAGAGCC
TTGAATGGATTGGAGGCATTAATCCTAATGGTGGTACTACCTATAATCAGAAGTTCAAGGGCAAGGC
CACATTGACTATTGACAAGTCCTCCAGCTCAGCCTTCATGGAGCTCCGCAGCCTGACATCTGAGGAT
TCTGCAGTCTATTACTTTGCAACAGGTTGGGGACCTGACTACTGGGGCCAAGGCACCACTCTCACA
GTCTCCTCAGCCAAAACAACACCCCCATCAGTCTATCCACTGGCCCCT

| Framework 1 | CDR H1 | Framework 2 | CDR H2 |
|---|---|---|---|

EVQLQQSGPELVKPGASVKISCKTSGYTFTEYTVHWVKQSHGKSLEWIGGINPNGGTTYNQKFKGKATLT

| Framework 3 | CDR H3 | Framework 4 |
|---|---|---|

IDKSSSSAFMELRSLTSEDSAVYYFATGWGPDYWGQGTTLTVSSAKTTPPSVYPLAP

**B**

| VL: nucleotide (SEQ ID NO:2) and amino acid (SEQ ID NO:4) sequence |
|---|

GATATTGTGCTGACACAATCTCCACTCACTTTGTCGGTTAACATTGGTCAACCAGCCTCTATCTCTTG
CAAGTCAAGTCAGAGCCTCTTATATACTAATGGAAAAACCTATTTTAATTGGTTATTACAGAGGCCAG
GCCAGTCTCCAAAGCGCCTAATCTATCTGGTGTCTAAACTGGACTCTGGAGTCCCTGACAGGTTCAC
TGGCAGTGGATCAGGAACAGATTTTACACTGAAAATCAGCAGAGTGGAGGCTGAAGATTTGGGAGTT
TATTACTGCGCGCAAGGTACACATTTTCCGTGGACGTTCGGTGGAGGGACCAAGCTGGAAATCAAA
CGGGCTGATGCTGCACCAACTGTATCC

| Framework 1 | CDR L1 | Framework 2 | CDR L2 |
|---|---|---|---|

DIVLTQSPLTLSVNIGQPASISCKSSQSLLYTNGKTYFNWLLQRPGQSPKRLIYLVSKLDSGVPDRFTGS

| Framework 3 | CDR L3 | Framework 4 |
|---|---|---|

GSGTDFTLKISRVEAEDLGVYYCAQGTHFPWTFGGGTKLEIKRADAAPTVS

FIG. 3

| Nuclotide sequence (SEQ ID NO:5) of the construct VH-linker-VL relative to the scFv-hERG1 |
|---|
| GAGGTCCAACTGCAACAGTCTGGACCTGAACTGGTGAAGCCTGGGGCTTCTGTGAAGATATCCTGCAAGACTTCAGGA TACACATTCACTGAATACACCGTTCACTGGGTGAAACAGAGCCATGGAAAGAGCCTTGAATGGATTGGAGGCATTAAT CCTAATGGTGGTACTACCTATAATCAGAAGTTCAAGGGCAAGGCCACATTGACTATTGACAAGTCCTCCAGCTCAGCC TTCATGGAGCTCCGCAGCCTGACATCTGAGGATTCTGCAGTCTATTACTTTGCAACAGGTTGGGGACCTGACTACTGG GGCCAAGGCACCACTCTCACAGTCTCCTCAGCCAAAACAACACCCCCATCAGTCTATCCACTGGCCCCTGG<u>CTCGAG</u> TGGTGGAGGCGGTTCAGGCGGAGGTGGCTCTGGCGGTA<u>GTGCAC</u>TGGATATTGTGCTGACACAATCTCCACTC ACTTTGTCGGTTAACATTGGTCAACCAGCCTCTATCTCTTGCAAGTCAAGTCAGAGCCTCTTATATACTAATGGAAAA ACCTATTTTAATTGGTTATTACAGAGGCCAGGCCAGTCTCCAAAGCGCCTAATCTATCTGGTGTCTAAACTGGACTCT GGAGTCCCTGACAGGTTCACTGGCAGTGGATCAGGAACAGATTTTACACTGAAAATCAGCAGAGTGGAGGCTGAAGAT TTGGGAGTTTATTACTGCGCGCAAGGTACACATTTTCCGTGGACGTTCGGTGGAGGGACCAAGCTGGAAATCAAACGG GCTGATGCTGCACCAACTGTATCC |

FIG. 4

| Aminoacid sequence (SEQ ID NO:6) of the construct VH-linker-VL relative to the scFv-hERG1 |
|---|

Framework H1                  CDR H1     Framework H2    CDR H2

EVQLQQSGPELVKPGASVKIS<u>CKTS</u>GYTFTEYTVH<u>WVKQSHGKSLEWI</u>GGINPNGGTTYNQKFKGKA

     Framework H3               CDR H3    Framework 4H       Linker

TLTIDKSSSSAFMELRSLTSEDSAVYY<u>FATGWGPDY</u>WGQGTTLTVSSAKTTPPSVYPLAP***GSSGGGGSGGGGS***

       Framework L1            CDR L1          Framework L2

***GGSAL***DIVLTQSPLTLSVNIGQPASIS<u>CKSSQSLLYTNGKTYFN</u>WLLQRPGQSPKRLIY<u>LVSK</u>

     Framework L3                CDR L3   Framework L4

LDS<u>GVPDRFTGSGSGTDFTLKISRVEAEDLGVYYC</u>AQGTHFPWTF<u>GGGTKLEIKR</u>ADAAPTVS

FIG. 5

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- IT 1367861

### Non-patent literature cited in the description

- **HOLLIGER P ; HUDSON PJ.** Engineered antibody fragments and the rise of single domains. *Nature Biotechnology,* 2005, vol. 23, 1126-1136
- **HENG CK ; OTHMAN RY.** Bioinformatics in molecular immunology laboratories demonstrated: Modeling an anti-CMV scFv antibody. *Bioinformation,* 2006, vol. 1, 118-120
- **SHEN, Z ; YAN H ; ZHANG Y ; MERNAUGH RL ; ZENG X.** Engineering peptide linkers for scFv immunosensors. *Anal. Chem.,* 2008, vol. 80, 1910-1917
- **ARGOS, P.** An investigation of oligopeptides linking domains in protein tertiary structures and possible candidates for general gene fusion. *J Mol Biol.,* 1990, vol. 211, 943-958
- **HUSTON, J. S. ; D. LEVINSON ; M. MUDGETT-HUNTER ; M. S. TAI ; J. NOVOTNY ; M. N. MARGOLIES ; R. J. RIDGE ; R. E. BRUCCOLERI ; E. HABER ; R. CREA et al.** Protein engineering of antibody binding sites: recovery of specific activity in an anti-digoxin single-chain Fv analogue produced in Escherichia coli. *Proc. Natl. Acad. Sci. USA,* 1988, vol. 85, 5879-5883
- **KORTT AA ; DOLEZAL O ; POWER BE ; HUDSON PJ.** Dimeric and trimeric antibodies: high avidity scFvs for cancer targeting. *Biomol Eng.,* 2001, vol. 18, 95-108
- **JAIN RK.** Transport of molecules across tumor vasculature. *Cancer Metastasis Rev.,* 1987, vol. 6, 559-593
- **ARCANGELI A ; BECCHETTI A.** 2010 New trends in cancer therapy: targeting ion channels and transporters. *Pharmaceuticals,* vol. 3, 1202-1224
- **WANG, Z. ; RAIFU M ; HOWARD M ; SMITH L ; HANSEN D ; GOLDSBY R ; RATNER D.** Universal PCR amplification of mouse immunoglobulin gene variable regions: the design of degenerate primers and an assessment of the effect of DNA polymerase 3' to 5' exonuclease activity. *J. Immunol. Methods,* 2000, vol. 233, 167-177
- **HOOGENBOOM HR ; VOLCKAERT G ; RAUS JC.** Construction and expression of antibody-tumor necrosis factor fusion proteins. *Mol. Immunol,* 1991, vol. 28, 1027-1037
- **KIM BY ; RUTKA JT ; CHAN WC.** *Nanomedicine. N. Engl. J. Med.,* 2010, vol. 363, 2434-2443
- **CLARK M.** Antibody humanization: a case of the 'Emperor's new clothes'?. *Immunol Today,* 2000, vol. 21, 397-402